**Europäisches Patentamt**

**European Patent Office**

**Office européen des brevets**

(19)

(11) Veröffentlichungsnummer: **0 082 342**
**B1**

(12) # EUROPÄISCHE PATENTSCHRIFT

(45) Veröffentlichungstag der Patentschrift:
**29.04.87**

(51) Int. Cl.⁴: **B 01 J 27/10,** C 07 C 17/02,
C 07 C 19/045, B 01 J 31/30

(21) Anmeldenummer: **82110897.4**

(22) Anmeldetag: **25.11.82**

(54) **Katalysatorgemisch und Verfahren zur Herstellung von 1,2-Dichlorethan.**

(30) Priorität: **08.12.81 DE 3148450**

(43) Veröffentlichungstag der Anmeldung:
**29.06.83 Patentblatt 83/26**

(45) Bekanntmachung des Hinweises auf die Patenterteilung:
**29.04.87 Patentblatt 87/18**

(84) Benannte Vertragsstaaten:
**BE CH DE FR GB IT LI NL SE**

(56) Entgegenhaltungen:
**DE-A-2 427 045**
**DE-A-2 540 291**
**FR-A-2 119 403**
**US-A-2 486 379**
**US-A-4 058 574**
**US-A-4 256 719**
**US-A-4 282 165**

(73) Patentinhaber: **HOECHST AKTIENGESELLSCHAFT,**
**Postfach 80 03 20, D-6230 Frankfurt am Main 80**
**(DE)**

(72) Erfinder: **Hundeck, Joachim, Dr., Argelander**
**Strasse 135, D-5300 Bonn 1 (DE)**
Erfinder: **Scholz, Harald, Dr., Am Beissel 8, D-5042**
**Erftstadt 12 (DE)**
Erfinder: **Hennen, Hans, Kendenicher Strasse 86,**
**D-5030 Hürth (DE)**

EP 0 082 342 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

# 0 082 342

## Beschreibung

Die Herstellung von 1,2-Dichlorethan durch Reaktion von Ethylen mit Chlor in 1,2-Dichlorethan als Lösungsmittel und Reaktionsmedium ist bekannt. Als hauptsächliches Nebenprodukt fällt bei dieser Reaktion 1,1,2-Trichlorethan durch Substitution von Dichlorethan an. Zur Unterdrückung dieser Substitutionsreaktion verwendet man als Katalysatoren neben den Chloriden der Elemente der IV. bis VI. Gruppe des Periodensystems vor allem wasserfreies Eisen-III-chlorid, zum Teil bei gleichzeitiger Gegenwart von Sauerstoff, da Eisen-III-chlorid leicht zugänglich und preisgünstig ist.

Das gebildete rohe, katalysatorhaltige Dichlorethan wird im allgemeinen aus dem Reaktionsgefäß abgezogen und zur Entfernung des Katalysators und des im Rohprodukt enthaltenen Chlorwasserstoffes mit Wasser bzw. wäßrigen Alkalilösungen behandelt und anschließend nach bekannten Verfahren destillativ aufgearbeitet.

Die Verwendung von $FeCl_3$ als Katalysator bei der Additionschlorierung von Ethylen ist mit gewissen Nachteilen verbunden. So wirkt $FeCl_3$ in Gegenwart von Wasser korrosiv gegenüber den metallischen Werkstoffen von Reaktoren, Kolonnen oder Wärmeaustauschern soweit diese mit dem Katalysator in Berührung kommen. Das zur Chlorierung normalerweise verwendete Chlor mit technischem Reinheitsgrad enthält stets Spuren von Feuchtigkeit sowie Chlorwasserstoff aus unerwünschten Nebenreaktionen.

Sofern die bei der Chlorierung von Ethylen freigesetzte Wärmeenergie nutzbar gemacht werden soll, muß die Reaktion bei Temperaturen oberhalb des Siedepunktes von Dichlorethan bei Normaldruck durchgeführt werden. Da mit steigender Temperatur die Korrosionsrate erheblich zunimmt, ist es unumgänglich, die Apparaturen für die Chlorierungsreaktion mit korrosionsfesten Werkstoffen auszustatten, wodurch die Wirtschaftlichkeit des angewandten Verfahrens beeinträchtigt wird.

Es wurde nunmehr gefunden, daß die durch $FeCl_3$ als Katalysator bei der Herstellung von 1,2-Dichlorethan verursachte Korrosion in nicht korrosionsfesten Reaktoren erheblich vermindert werden kann, wenn man den $FeCl_3$-Katalysator mit bestimmten Zusätzen beaufschlagt. Darüberhinaus wurde festgestellt, daß diese Zusätze sich auch vorteilhaft auf die Nebenproduktbildung, welche dadurch verringert wird, auswirken.

Gegenstand der Erfindung ist somit ein Katalysatorgemisch, bestehend aus wasserfreiem Eisen-III-chlorid sowie einer weiteren Gemischkomponente, zur Herstellung von 1,2-Di-chlorethan durch Reaktion vcn Ethylen mit Chlor in einem Lösungsmittel bei Normal- oder Überdruck, welches dadurch gekennzeichnet ist, daß die weitere Gemischkomponente eine in Bezug auf die Eisen-III-chlorid-Menge etwa äquivalente Menge einer Stickstoffbase oder ein Salz dieser Base ist.

Die Stickstoffbase kann $NH_3$, ein primäres, sekundäres oder tertiäres Alkyl-, Aralkyl-, Aryl- oder alicyclisches Amin oder ein Polyamin sein. Das Salz der Stickstoffbase ist vorzugsweise ein Halogensalz, beispielsweise Ammoniumchlorid.

Ein weiterer Gegenstand der Erfindung ist ein Verfahren zur Herstellung von 1,2-Dichlorethan unter Verwendung des erfindungsgemäßen Katalysatorgemisches.

Das Verfahren der Erfindung zur Herstellung von 1,2-Dichlorethan durch Reaktion von Ethylen mit Chlor in einem Lösungsmittel in Gegenwart eines Katalysatorgemisches aus wasserfreiem Eisen-III-chlorid und einer weiteren Gemischkomponente sowie gegebenenfalls eines Inhibitors zur Verhinderung der Nebenproduktbildung bei einer Temperatur von etwa 20 bis 200°C und Normaloder Überdruck und Abtrennen des 1,2-Dichlorethans aus dem Chlorierungsgemisch durch Destillation ist dadurch gekennzeichnet, daß

a) die weitere Gemischkomponente eine Stickstoffbase oder ein Salz dieser Base ist,

b)  die weitere Gemischkomponente in etwa äquivalenter Menge zur Eisen-III-chlorid-Menge im Gemisch vorliegt und

c) die Konzentration des Eisen-III-chlorids 0,003 bis etwa 0,3 Gew %, bezogen auf die Menge des Lösungsmittels, beträgt.

Wie bereits in Erläuterung des Katalysatorgemisches erwähnt, kann die Stickstoffbase $NH_3$, ein primäres, sekundäres oder tertiäres Alkyl-, Aralkyl-, Aryl- oder alicyclisches Amin oder ein Polyamin sein. Das Salz der Stickstoffbase ist vorzugsweise ein Halogensalz, insbesonders Ammoniumchlorid.

Gemäß einer bevorzugten Ausführungsform des Verfahrens der Erfindung wird als Lösungsmittel 1,2-Dichlorethan und als Inhibitor Sauerstoff verwandt.

Im einzelnen ist zum Verfahren der Erfindung noch folgendes zu bemerken:

Der erfindungsgemäße Katalysator wird im allgemeinen in dem im Reaktor vorgelegten Lösungsmittel gelöst bzw. suspendiert. Er kann aber auch außerhalb der Reaktionslösung zubereitet werden, indem das wasserfreie $FeCl_3$ zusammen mit der weiteren Katalysatorkomponente in beispielsweise 1,2-Dichlorethan suspendiert wird und die Suspension in den Reaktor eingebracht wird. Es ist ferner möglich, das im Reaktor vorgelegte Lösungsmittel mit wasserfreiem $FeCl_3$ und $NH_3$ oder einem Amin zu beschicken. Bei der anschließenden Reaktion entsteht dann genügend Chlorwasserstoff zur Bildung des entsprechenden Ammoniumsalzes.

Der erfindungsgemäße Katalysator ist als technisch fortschrittlich zu betrachten, da durch ihn die bei den bekannten Verfahren zur Herstellung von 1,2-Dichlorethan auftretende und nachteilig sich auswirkende Korrosion bei Verwendung von nicht korrosionsbeständigen, metallischen Reaktoren in einem erheblichen Maße zurückgedrängt wird. Weiterhin wurde festgestellt, daß mit Ausnahme von geringen Mengen des ersten Substitutionsproduktes 1,1,2-Trichlorethan und einer entsprechend geringen Menge Chlorwasserstoff unter den erfindungsgemäßen Verfahrensbedingungen so gut wie keine weiteren Nebenprodukte gebildet werden. Die Reaktionslösung bleibt auch bei längerer Reaktionsdauer hell, wenn die erfindungsgemäßen Zusätze in dem vorhandenen Eisenchlorid annähernd äquivalenter Menge in der Reaktionslösung vorliegen. Ein gegebenenfalls im Zuge der Reaktion bereits dunkel gefärbtes Reaktionsgemisch hellt sich im Ausmaß der Zugabe der genannten Verbindungen beim weiteren Reaktionsverlauf wieder auf. Schließlich ist festzustellen, daß beim Verfahren der Erfindung der Umsatz bei hoher Raum/Zeit-Ausbeute nahezu quantitativ ist.

Das Verfahren der Erfindung kann beispielsweise in dem in der DE-A-24 27 043 beschriebenen Schlaufenreaktor oder in jedem anderen für die Durchführung des Verfahrens geeigneten Reaktor ausgeführt werden.

Der Gegenstand der Erfindung sei anhand der nachfolgenden Beispiele näher erläutert:

**Beispiel 1**

In einem Schlaufenreaktor mit einem Fassungsvermögen von etwa 2 l wurden 2,0 kg 1,2-Dichlorethan sowie 4 g wasserfreies Eisen-III-chlorid vorgelegt. In dieses Gemisch wurden 0,42 g Ammoniak in Form einer 0,67 Gew%igen Lösung in Dichlorethan bei 30-40° C eingebracht. Der aufsteigende Teil der Reaktorschlaufe enthielt eine Füllkörperschicht. Unterhalb der Füllkörperschicht befanden sich Einleitungsrohre in den Reaktor für Ethylen, Chlor und Luft, durch die je ca. 60 l/h Chlor und Ethylen sowie 13 l/h Luft eingespeist wurden. Die Reaktorflüssigkeit wurde im Reaktorsystem nach dem Mammutpumpenprinzip umgewälzt und dabei die Katalysatormischung gleichmäßig in der flüssigen Phase suspendiert. Während der Reaktion stellte sich im Reaktionsgemisch eine Reaktionstemperatur von etwa 77°C ein. Die Konzentration des in der Reaktorflüssigkeit gelösten Katalysatorgemisches, bestimmt als $FeCl_3$, betrug nach Verlauf mehrerer Tage 0,13 Gew%.

In einem über dem Reaktor angeordneten Wasserkühler wurden die aus dem Reaktor abziehenden Dichlorethan-Dämpfe kondensiert und anschließend ein der produzierten Menge entsprechender Teil des Kondensates mittels eines Kondensatteilers abgezweigt und entnommen, während überschüssiges Kondensat in die Reaktionszone zurückgeleitet wurde. Mittels einer Kühlfalle wurde ein weiterer Dichlorethan-Anteil aus dem überwiegend aus Inertgasen bestehenden Abgas abgeschieden. Nach einer Laufzeit des kontinuierlichen Verfahrens von mehreren Tagen hatte sich das Katalysatorgemisch in der Reaktorflüssigkeit weitgehend aufgelöst, und eine kolorimetrische Bestimmung des Fe-Gehaltes in der Reaktorflüssigkeit ergab einen $FeCl_3$-Gehalt von ca. 0,13 Gew%. Die stündlich anfallende Menge an 1,2-Dichlorethan betrug 262 g. Das Verfahren wurde kontinuierlich über 14 Tage durchgeführt.

Die Analyse des im Kondensator anfallenden Produktes A bzw. der Reaktorflüssigkeit B nach Beendigung der Reaktion ergab folgende Werte:

|  | Produkt A (Gew%) | Produkt B (Gew%) |
|---|---|---|
| $C_2H_5Cl$ | < 0,002 | < 0,002 |
| 1,2-EDC | 99,94 | 99,82 |
| 1,1,2-ETC | 0,04 | 0,14 |
| HCl | 0,001 | — |
| Sonstige | 0,01 | 0,04 |

EDC = 1,2-Dichlorethan
ETC = 1,1,2-Trichlorethan

**Beispiel 2**

Es wurde analog Beispiel 1 verfahren, wobei jedoch zusätzlich in das im Reaktor umgewälzte Reaktionsgemisch stündlich 30 ml 1,2-Dichlorethan mit einem Gehalt von 0,4 Gew% 1,1,2-Trichlorethan mittels eines Tropftrichters zugetropft wurden. Die stündlich anfallende Menge an 1,2-Dichlorethan betrug 326 g. Die Versuchsdauer betrug 8 Tage. Die Analyse des im Kondensator anfallenden Produktes A bzw. der Reaktorflüssigkeit B nach Beendigung der Reaktion ergab folgende Werte:

|  | Produkt A (Gew%) | Produkt B (Gew%) |
|---|---|---|
| $C_2H_5Cl$ | < 0,002 | < 0,002 |
| 1,2-EDC | 99,87 | 99,61 |
| 1,1,2-ETC | 0,10 | 0,34 |
| HCl | 0,001 | — |
| Sonstige | 0,03 | 0,05 |

**Beispiel 3**

Es wurde analog Beispiel 1 verfahren, wobei jedoch die Konzentration des $FeCl_3$ im Reaktionsgemisch sowie das Molverhältnis von $FeCl_3$ zum Ammoniak variiert wurden. In welchem Umfang die vorgenommenen Änderungen erfolgten und wie diese Änderungen sich auf die Gehalte von 1,1,2-ETC und HCl in dem im Kondensator anfallenden Produkt auswirkten, ist aus der nachfolgenden Tabelle ersichtlich.

| Konzentration $FeCl_3$ (Gew%) | Molverhältnis $FeCl_3:NH_3$ | Gew% 1,1,2-ETC | Gew% HCl |
|---|---|---|---|
| 0,07 | 1 : 2 | 0,2 | 0,004 |
| 0,34 | 1 : 2 | 0,6 | 0,002 |
| 0,45 | 1 : 1,5 | 0,1 | 0,001 |
| 0,32 | 1 : 1 | 0,06 | < 0,001 |

Die stündlich anfallende Menge an 1,2-Dichlorethan betrug für den Versuch mit der $FeCl_3$-Konzentration von 0,32 Gew% 260 g und die Versuchsdauer 19 Tage. Die Analyse des im Kondensator anfallenden Produktes A bzw. der Reaktorflüssigkeit B nach Beendigung der Reaktion ergab folgende Werte:

|  | Produkt A (Gew%) | Produkt B (Gew%) |
|---|---|---|
| $C_2H_5Cl$ | 0,004 | 0,006 |
| 1,2-EDC | 99,93 | 99,74 |
| 1,1,2-ETC | 0,06 | 0,23 |
| HCl | 0,002 | 0,03 |
| Sonstige | 0,003 | — |

**Beispiel 4**

Es wurde analog Beispiel 1 verfahren, wobei jedoch 1,35 kg 1,2-Dichlorethan eingesetzt und anstelle von Ammoniak 1,3 g Trimethylamin, gelöst in 30 ml 1,2-Dichlorethan, der Reaktionslösung zugefügt wurden. Die stündlich anfallende Menge an 1,2-Dichlorethan betrug 276 g und die Versuchsdauer 6 Tage. Weiterhin betrug der in der Lösung kolorimetrisch ermittelte $FeCl_3$-Gehalt im Mittel 0,13 Gew%.
Die Analyse des im Kondensator anfallenden Produktes A nach Beendigung der Reaktion ergab folgende Werte:

|  | Produkt A (Gew%) |
|---|---|
| $C_2H_5Cl$ | — |
| 1,2-EDC | 99,86 |
| 1,1,2-ETC | 0,13 |
| HCl | 0,01 |
| Sonstige | 0,006 |

4

**Beispiel 5**

Es wurde analog Beispiel 4 verfahren, wobei jedoch als Katalysator 1,7 g $FeCl_3$ sowie 0,65 g Diaminoethan der Reaktionslösung zugefügt wurden. Der kolorimetrisch ermittelte Gehalt an $FeCl_3$ in der Lösung betrug im Mittel 0,07 Gew%. Stündlich wurden 268 g Dichlorethan erhalten und der Versuch dauerte über 3 Tage an.

Die Analyse des im Kondensator anfallenden Produktes A ergab folgende Werte:

| | Produkt A (Gew%) |
|---|---|
| $C_2H_5Cl$ | — |
| 1,2-EDC | 99,13 |
| 1,1,2-ETC | 0,85 |
| HCl | 0,02 |
| Sonstige | 0,01 |

**Beispiel 6**

Es wurde analog Beispiel 1 verfahren, wobei jedoch 1,5 kg 1,2-Dichlorethan eingesetzt wurden und die Katalysatormenge 3,3 g $FeCl_3$ und 3,0 g Triethanolamin betrug. Der in der Reaktionslösung kolorimetrisch ermittelte $FeCl_3$-Gehalt betrug im Mittel 0,25 Gew%. Stündlich wurden 268 g Dichlorethan erhalten und der Versuch dauerte über 6 Tage.

Die Analyse des im Kondensator anfallenden Produktes A ergab folgende Werte:

| | Produkt A (Gew%) |
|---|---|
| $C_2H_5Cl$ | < 0,002 |
| 1,2-EDC | 99,65 |
| 1,1,2-ETC | 0,33 |
| HCl | 0,007 |
| Sonstige | 0,01 |

**Beispiel 7**

a) In einem 2 l-Rundkolben, welcher mit Rührer, Tropftrichter und Rückflußkühler ausgestattet war, wurden 2 kg 1,2-Dichlorethan mit 2,1 g Eisen-III-chlorid vorgelegt. Die Mischung wurde unter Rühren zum Sieden erhitzt und danach 0,2 g Ammoniak, gelöst in 58 g Dichlorethan eingetropft, so daß die Gesamtkatalysatormenge 2,3 g betrug. Nachdem das Gemisch weitere 5 Stunden unter Rückfluß gekocht worden war, wurde kolorimetrisch ein Gehalt von 0,11 Gew% $FeCl_3$ in der Lösung ermittelt.

b) Das Gemisch aus Dichlorethan und Katalysator wurde anschließend in den gemäß Beispiel 1 beschriebenen Schlaufenreaktor eingefüllt und danach je etwa 60 l/h Chlor und Ethylen zusammen mit ca. 5 l/h Luft in den Reaktor eingeleitet. Stündlich wurden 273 g Dichlorethan erhalten und der Versuch dauerte 8 Tage an.

Die Analyse des im Kondensator anfallenden Produktes A ergab folgende Werte:

| | Produkt A (Gew%) |
|---|---|
| $C_2H_5Cl$ | < 0,002 |
| 1,2-EDC | 99,51 |
| 1,1,2-ETC | 0,48 |
| HCl | 0,002 |
| Sonstige | 0,009 |

**Beispiel 8**

a) Es wurde zunächst analog Beispiel 7a) verfahren, wobei jedoch 1,5 kg 1,2-Dichlorethan mit 12 g FeCl₃ unter Rühren zum Sieden erhitzt wurden. In das Gemisch wurde eine Lösung von 2,7 g Chlorwasserstoff in 750 g Dichlorethan und anschließend eine Lösung von 1,26 g $NH_3$ in 273 g Dichlorethan eingetropft. Das Reaktionsgemisch wurde nach Abkühlen filtriert und der Filterrückstand getrocknet. Es wurden 14,4 g trockener Katalysator erhalten.

b) Zur Herstellung von 1,2-Dichlorethan wurden 4 g des gemäß a) hergestellten Katalysators sowie 2 g FeCl₃ in 2,7 kg 1,2-Dichlorethan suspendiert und nach Einengen des Gemischvolumens auf etwa 2 Liter das Gemisch in den in Beispiel 1 beschriebenen Schlaufenreaktor eingebracht. Stündlich wurden in den Schlaufenreaktor jeweils 60 l Chlor und Ethylen sowie 15 l Luft eingeleitet und die Reaktion unter den in Beispiel 1 genannten Reaktionsbedingungen in Gang gebracht. Es wurden 266 g 1,2-Dichlor-ethan erhalten und der Versuch dauerte 6 Tage an. Der in der Reaktionslösung kolorimetrisch ermittelte FeCl₃-Gehalt betrug im Mittel 0,15 Gew%.

Die Analyse des im Kondensator anfallenden Produktes A bzw. der Reaktorflüssigkeit B nach Beendigung der Reaktion ergab folgende Werte:

|           | Produkt A (Gew%) | Produkt B (Gew%) |
|-----------|------------------|------------------|
| $C_2H_5Cl$ | < 0,002          | < 0,002          |
| 1,2-EDC   | 99,93            | 99,78            |
| 1,1,2-ETC | 0,06             | 0,19             |
| HCl       | 0,001            | —                |
| Sonstige  | 0,01             | 0,03             |

**Beispiel 9**

In einem Reaktor zur technischen Herstellung von 1,2-Dichlorethan wurden an 4 Stellen des Reaktors Korrosionsproben eingesetzt und die Proben den Verfahrensbedingungen gemäß Erfindung ausgesetzt. Im Abstand von 20 Tagen wurden die Proben aus dem Reaktor entnommen und der durch Korrosion bedingte Abtrag ermittelt. Bei Verwendung des erfindungsgemäßen FeCl₃/NH₃-Katalysators und Einhaltung einer Reaktionstemperatur von 100 - 110°C, betrug die durchschnittliche Korrosionsrate für unlegierten Stahl weniger als 0,05 mm pro Jahr. Wurde dagegen das Verfahren in bekannter Weise unter Verwendung von ausschließlich FeCl₃ als Katalysator durchgeführt, so betrug die durchschnittliche Korrosionsrate für unlegierten Stahl 0,43 mm pro Jahr.

**Patentansprüche**

1) Katalysatorgemisch, bestehend aus wasserfreiem Eisen-III-chlorid sowie einer weiteren Gemischkomponente, zur Herstellung von 1,2-Dichlorethan durch Reaktion von Ethylen mit Chlor in einem Lösungsmittel bei Normal- oder Überdruck, dadurch gekennzeichnet, daß die weitere Gemischkomponente eine in Bezug auf die Eisen-III-chlorid-Menge etwa äquivalente Menge einer Stickstoffbase oder ein Salz dieser Base ist.

2) Gemisch nach Anspruch 1, dadurch gekennzeichnet, daß die Stickstoffbase NH₃, ein primäres, sekundäres oder tertiäres Alkyl-, Aralkyl-, Aryl- oder ein alicyclisches Amin oder ein Polyamin ist.

3) Gemisch nach Anspruch 1, dadurch gekennzeichnet, daß das Salz der Stickstoffbase ein Halogensalz ist.

4) Gemisch nach Anspruch 1, dadurch gekennzeichnet, daß das Salz der Stickstoffbase Ammoniumchlorid ist.

5) Verfahren zur Herstellung von 1,2-Dichlorethan durch Reaktion von Ethylen mit Chlor in einem Lösungsmittel in Gegenwart eines Katalysatorgemisches aus wasserfreiem Eisen-III-chlorid und einer weiteren Gemischkomponente sowie gegebenenfalls eines Inhibitors zur Verhinderung der Nebenproduktbildung bei einer Temperatur von etwa 20 bis 200° C und Normaloder Überdruck und Abtrennen des 1,2-Dichlorethans aus dem Chlorierungsgemisch durch Destillation dadurch gekennzeichnet, daß

a) die weitere Gemischkomponente eine Stickstoffbase oder ein Salz dieser Base ist,

b) die weitere Gemischkomponente in etwa äquivalenter Menge zur Eisen-III-chlorid-Menge im Gemisch vorliegt und

c) die Konzentration des Eisen-III-chlorids 0,005 bis etwa 0,5 Gew%, bezogen auf die Menge des Lösungsmittels, beträgt.

6) Verfahren nach Anspruch 5, dadurch gekennzeichnet, daß die Stickstoffbase NH₃, ein primäres, sekundäres oder tertiäres Alkyl-, Aralkyl-, Aryl- oder ein alicyclisches Amin oder ein Polyamin ist.

7) Verfahren nach Anspruch 5, dadurch gekennzeichnet, daß das Salz der Stickstoffbase ein Halogensalz ist.

6

8) Verfahren nach Anspruch 5, <u>dadurch gekennzeichnet</u>, daß das Salz der Stickstoffbase Ammoniumchlorid ist.

9) Verfahren nach Anspruch 5 - 8, <u>dadurch gekennzeichnet</u>, daß das Lösungsmittel 1,2-Dichlorethan ist.

10) Verfahren nach Anspruch 5 - 9, <u>dadurch gekennzeichnet</u>, daß der Inhibitor Sauerstoff ist.

## Claims

1. Catalyst mixture consisting of anhydrous iron(III) chloride and a further mixing component, for making 1,2-dichloroethane by subjecting ethylene to reaction with chlorine in a solvent at atmospheric or elevated pressure, characterized in that the further mixing component is a quantity approximately equivalent to the iron (III) chloride quantity, of a nitrogen base or a salt of that base.

2. Mixture as claimed in claim 1, wherein the nitrogen base is $NH_3$, a primary, secondary or tertiary alkyl, aralkyl, aryl amine or alicyclic amine or a polyamine.

3. Mixture as claimed in claim 1, wherein the salt of the nitrogen base is a halogen salt.

4. Mixture as claimed in claim 1, wherein the salt of the nitrogen base is ammonium chloride.

5. Process for making 1,2-dichloroethane by subjecting ethylene to reaction with chlorine in a solvent in the presence of a catalyst mixture consisting of anhydrous iron(III)chloride and a further mixing component and, if desired, an agent inhibiting the formation of byproducts, at a temperature of about 20 to 200°C, at atmospheric or elevated pressure and distillatively separating the 1,2-dichloroethane from the chlorination mixture, characterized in that

a) the further mixing component is a nitrogen base or a salt of that base;

b) the further mixing component is used in the mixture in a quantity approximately equivalent to the quantity of iron(III)chloride, and

c) the iron(III)chloride concentration is 0.005 to about 0.5 weight %, based on the quantity of solvent.

6. Process as claimed in claim 5, wherein the nitrogen base is $NH_3$, a primary, secondary or tertiary alkyl, aralkyl, aryl amine or alicyclic amine or a polyamine.

7. Process as claimed in claim 5, wherein the salt of the nitrogen base is a halogen salt.

8. Process as claimed in claim 5, wherein the salt of the nitrogen base is ammonium chloride.

9. Process as claimed in any of claims 5 to 8, wherein the solvent is 1,2-dichloroethane.

10. Process as claimed in any of claims 5 to 9, wherein the inhibitor is oxygen.

## Revendications

1. Mélange catalytique, consistant en du chlorure de fer III anhydre ainsi qu'un composant supplémentaire, pour la préparation du 1,2-dichloroéthane par réaction de l'éthylène avec le chlore dans un solvant sous pression normale ou élevée, caractérisé en ce que le composant de mélange supplémentaire est une quantité à peu près équivalente, par rapport à la quantité de chlorure de fer III, d'une base azotée ou d'un sel de cette base.

2. Mélange selon la revendication 1, caractérisé en ce que la base azotée est $NH_3$, une alkylamine, une arylalkylamine, une arylamine ou une amine alicyclique primaire, secondaire ou tertiaire ou une polyamine.

3. Mélange selon la revendication 1, caractérisé en ce que le sel de la base azotée est un sel halogène.

4. Mélange selon la revendication 1, caractérisé en ce que le sel de la base azotée est le chlorure d'ammonium.

5. Procédé de préparation du 1,2-dichloroéthane par reaction de l'éthylène avec le chlore dans un solvant en présence d'un mélange catalytique de chlorure de fer III anhydre et d'un composant de mélange supplémentaire et, éventuellement, d'un inhibiteur destiné à empêcher la formation de sous-produits, à une température d'environ 20 à 200°C sous pression normale ou sous pression élevée et séparation du 1,2-dichloroéthane du mélange de chloration par distillation, caractérisé en ce que

a) le composant de mélange supplémentaire est une base azotée ou un sel de cette base,

b) le composant de mélange supplémentaire est présent dans le mélange en quantité à peu près équivalente à la quantité de chlorure de fer III et

c) la concentration du chlorure de fer III est d'environ 0,005 à 0,5 % en poids, par rapport à la quantité de solvant.

6. Procédé selon la revendication 5, caractérisé en ce que la base azotée est $NH_3$, une alkylamine, une arylalkylamine, une arylamine ou une amine alicyclique primaire, secondaire ou tertiaire ou une polyamine.

7. Procédé selon la revendication 5, caractérisé en ce que le sel de la base azotée est un sel halogené.

8. Procédé selon la revendication 5, caractérisé en ce que le sel de la base azotée est le chlorure d'ammonium.

9. Procédé selon l'une des revendications 5 à 8, caractérisé en ce que le solvant est le 1,2-dichloroethane.

10. Procédé selon l'une des revendications 5 à 9, caractérisé en ce que l'inhibiteur est l'oxygène.